(19) Europäisches Patentamt
European Patent Office
Office européen des brevets

(11) Veröffentlichungsnummer: **0 062 284**
**B1**

# (12) EUROPÄISCHE PATENTSCHRIFT

(45) Veröffentlichungstag der Patentschrift: **27.06.84**

(51) Int. Cl.³: **C 07 D 473/12**

(21) Anmeldenummer: **82102666.3**

(22) Anmeldetag: **30.03.82**

## (54) Verfahren zur Herstellung von Coffein.

(30) Priorität: **07.04.81 DE 3113880**

(43) Veröffentlichungstag der Anmeldung: **13.10.82 Patentblatt 82/41**

(45) Bekanntmachung des Hinweises auf die Patenterteilung: **27.06.84 Patentblatt 84/26**

(84) Benannte Vertragsstaaten: **DE FR GB IT**

(56) Entgegenhaltungen:
**US - A - 1 865 378**
**US - A - 2 509 084**
**US - A - 2 523 496**

(73) Patentinhaber: **BASF Aktiengesellschaft, Carl-Bosch-Strasse 38, D-6700 Ludwigshafen (DE)**

(72) Erfinder: **Bott, Kaspar, Dr., Rieslingweg 4, D-6706 Wachenheim (DE)**

EP 0 062 284 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein Verfahren zur Herstellung von Coffein.

In der Technik wird Coffein im allgemeinen dadurch gewonnen, daß man das Natrium- oder Kaliumsalz des Theophyllins mit Methylierungsmitteln reagieren läßt. Hierbei werden als Methylierungsmittel bevorzugt Dimethylsulfat und Methylchlorid eingesetzt (Kirk-Othmer, Encyclopedia of Chemical Technology, 2. Auflage, Band 3, Seite 916). Dimethylsulfat besitzt aber den Nachteil, daß es sehr toxisch ist und nur unter besonderen Vorsichtsmaßnahmen gehandhabt werden kann. Dieser Nachteil ist zwar bei der Verwendung von Methylchlorid weitgehend aufgehoben, doch muß man in diesem Falle ebenso wie beim Dimethylsulfat einen verhältnismäßig hohen Molüberschuß des Methylierungsmittels anwenden, um eine hohe Coffeinausbeute zu erhalten. Das bedeutet, daß ein hoher Anteil des Methylchlorids oder Dimethylsulfats durch die sogenannte »Leerverseifung« zu Methanol für die Coffein-Synthese verloren geht.

Weiterhin ist es erforderlich, die Methylierungskomponenten Methylchlorid und Dimethylsulfat in einer gesonderten Stufe aus Methanol und Chlorwasserstoff bzw. Schwefelsäure zu erzeugen.

Es wurde nun ein einfacheres Herstellungsverfahren für Coffein gefunden.

Gegenstand der Erfindung ist ein Verfahren zur Herstellung von Coffein, welches darin besteht, daß man Methanol und Kohlenmonoxid auf ein Alkalisalz des Theophyllins einwirken läßt.

Die dabei ablaufende Reaktion läßt sich durch die folgende Gleichung wiedergeben, in der M für ein Alkaliatom steht:

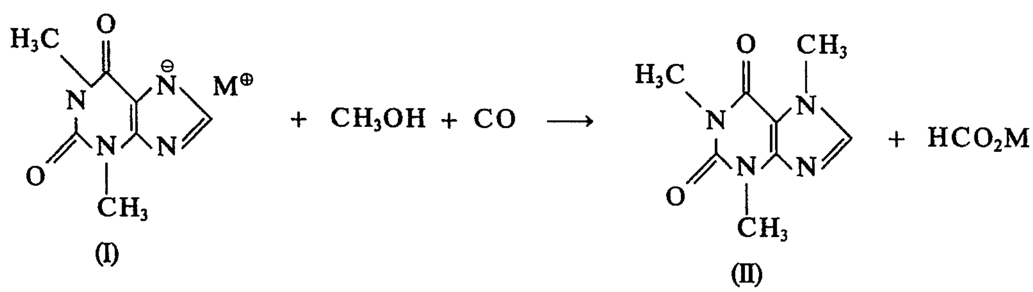


(I) (II)

Bei dieser Coffein-Synthese fällt als weiteres wertvolles Produkt Natriumformiat an, das technisch aus Natronlauge und Kohlenmonoxid gewonnen wird.

Zur Coffein-Synthese erhitzt man vorzugsweise das Natrium- oder Kaliumsalz des Theophyllins mit Kohlenmonoxid und einem Überschuß an Methanol auf 120 bis 170° C, vorzugsweise 130 bis 150° C. Der Kohlenmonoxiddruck im Reaktionsgefäß wird zweckmäßig auf 25 bis 100 bar, vorzugsweise auf 40 bis 60 bar eingestellt.

Bei der Umsetzung des Theophyllins auf dem erfindungsgemäßen Wege mit Methanol und Kohlenmonoxid reagiert ein Teil des im Überschuß eingesetzten Methanols mit Kohlenmonoxid. Das dabei gebildete, Methylformiat bedeutet aber keine Belastung für den Prozeß, weil auch Methylformiat anstelle der Mischung von Methanol und Kohlenmonoxid in die Methylierungsreaktion des Theophyllins eingesetzt werden kann. Im letzteren Fall ist es möglich, auf die Zuführung von Kohlenmonoxid ganz zu verzichten.

Durch das Verfahren wird die Herstellung von Coffein wesentlich vereinfacht. Darüber hinaus liefert es Coffein in hoher Ausbeute und guter Reinheit.

Die vorliegende Erfindung soll an den folgenden Versuchsbeispielen näher erläutert werden.

Beispiel 1

In einem 200-ml-Rührautoklaven wird eine Mischung aus 100 g Methanol und 22,6 g Kaliumsalz des Theophyllins 20 Stunden unter einem kontinuierlichen CO-Druck von 50 bar auf 140° C erhitzt. Man spült den Reaktorinhalt mit Methanol in einen Destillationskolben und destilliert das Methanol und das Methylformiat ab. Als fester Destillationsrückstand fallen 29,5 g einer Mischung von Coffein und Kaliumformiat an, die nach der Dünnschicht-Chromatographie nur noch 0,6% Theophyllin enthält. Aus dem Destillationsrückstand können mit Essigester 19,2 g (97%) Coffein extrahiert werden.

Beispiel 2

Eine Mischung aus 50 g Methanol, 50 g Methylformiat und 22,0 g Kaliumsalz des Theophyllins wird in einem Rührautoklaven 20 Stunden auf 140° C erhitzt. Man arbeitet wie in Beispiel 1 auf und erhält 29,7 g Destillationsrückstand, der aus 18,7 g Coffein, 0,18 g Theophyllin und 10 g Kaliumformiat zusammengesetzt ist. Die Coffeinausbeute beträgt 97,5%.

## Patentansprüche

1. Verfahren zur Herstellung von Coffein, dadurch gekennzeichnet, daß man bei 120—170°C ein Alkalisalz des Theophyllins mit einem Überschuß Methanol und mit Kohlenmonoxid umsetzt, wobei der Kohlenmonoxiddruck im Reaktionsgefäß auf 25 bis 100 bar eingestellt wird.

2. Verfahren gemäß Anspruch 1, dadurch gekennzeichnet, daß man das Methanol und Kohlenmonoxid durch Methylformiat ersetzt.

## Claims

1. A process for the preparation of caffeine, wherein an alkali metal salt of theophyllins is reacted with carbon monoxide and an excess of methanol at 120 to 170°C, the carbon monoxide pressure in the reaction vessel being adjusted to 25 to 100 bar.

2. A process as claimed in claim 1, wherein the methanol and carbon monoxide are replaced by methyl formate.

## Revendications

1. Procédé de préparation de caféine, caractérisé par le fait que l'on fait réagir, à 120—170°C, un sel alcalin de théophylline avec un excès de méthanol et avec de l'oxyde de carbone, la pression de l'oxyde de carbone dans le récipient de réaction étant réglée à une valeur de 25 à 100 bar.

2. Procédé selon la revendication 1, caractérisé par le fait que l'on remplace le méthanol et l'oxyde de carbone par du formiate de méthyle.